# EUROPEAN PATENT APPLICATION

(11) **EP 1 468 661 A1**
(43) Date of publication of application: **20.10.2004**
(21) Application number: 03008982.5
(22) Date of filing: 17.04.2003
(51) Int. Cl.: A61F 13/82, A61F 5/443

(54) **Disposable excreta device**

(71) Applicant: The Procter & Gamble Company, Cincinnati, Ohio 45202 (US)
(72) Inventor: Cinelli, Fabio, 65100 Pescara (IT); Denti, Federica, 65016 Montesilvana (IT); Costea, Karin, 67550 Worms (DE); Colaianni, Antonello, 65126 Pescara (IT); James, Martin, 65016 Montesilvana Colle (IT); Huhn, Wolfgang, 66013 Chieti (IT)
(74) Representative: Veronese, Pancrazio

(57) **Abstract**

The present invention relates to a disposable excreta management device comprising a composite body adhesive structure for releasable attachment of said device to the body of a wearer, said structure comprising a substrate and a layer of an adhesive material coated onto said substrate. The layer of adhesive material comprises a pattern of less adhesive areas where the adhesive is inactivated or covered by a second material having a lower adhesion on skin compared to said adhesive material. The device is easy to handle, not painful to remove, easy to reposition, and leaves no residues on skin.

## Description

### FIELD OF THE INVENTION

The present invention relates to a disposable excreta management device used for babies, children or adults. More particularly, the present invention relates to a disposable excreta management device comprising a composite body adhesive structure for releasable attachment to the body of a wearer.

### BACKGROUND

Excreta management devices are known as articles that are designed to collect body waste solids and fluids such as feces, urine, menstrual or other vaginal fluids. Such articles are manufactured with different structures and having different sizes and shapes according to their intended use.

Excreta management devices normally have a garment facing surface which usually consists of a water impervious backsheet and a body facing surface which has different forms according to the type of device. Often the device also comprises an absorbent core in between the topsheet and the backsheet, especially when it is meant to collect fluids.

In order to provide complete collection of fluids and solids, the prior art describes the use of so called body adhesives to attach the articles directly to the skin of the wearer in correspondence of an excretory orifice in the uro-genital area. In this manner the articles can be more effectively positioned so as to ensure direct collection of the discharged matter whilst being independent of the location of the undergarments.

For example GB 2,284,767 discloses sanitary napkins provided with a body adhesive to attach the article to the wearers' torso. WO 98/27918, WO 98/28023 and WO 98/28014 disclose absorbent articles having an adhesive applied to the body facing surface for securement of the article to the wearer without pain upon removal.

As an alternative in the art, an article for managing excreta has been developed which comprises a container in form of a bag for excreta containment having an adhesive flange for secure attachment to the body with a suitable body adhesive.

An adhesive flange used for an excreta management device conventionally has an opening positioned substantially at the center of the flange, and comprises a substrate continuously surrounding the opening and an adhesive layer supported by the substrate. The substrate to support the adhesive layer conventionally is made from substantially inelastic materials such as nonwoven materials, foams or plastic films. The adhesive layer is applied on the substrate such that the adhesive layer substantially continuously surrounds the opening. Therefore, the adhesive flange is adhesively attached to the skin in whole circumference of an excretory orifice when the device is worn. The complete attachment of the device to the desired area of the skin of a wearer is a key point in the excreta management device comprising an adhesive flange.

Such articles were originally designed as medical devices for the collection of urine or other discharges. Such excreta management devices are attached to the perineal area or urethral area of a wearer and are intended to entrap and immediately contain fecal material, urine and other bodily discharges. Representative excreta management device of his type are disclosed in, e.g. US 3,577,989 and GB 2,152,387.

Similar devices found application, with suitable structures and sizes also for light and moderate urinary incontinence, see e.g. those disclosed in EP 1,089,693 and EP 966,936, in which particularly the use of a flange having preferred e.g. oval shape in conjunction with a collection bag is disclosed.

Some examples of menstrual usage of a collection bag are disclosed in EP 1,104,666.

A not stable attachment of the device in the desired portion of the body leads to serious problems such as mispositioning of the article during use, as well as ineffective acquisition and/or incomplete containment of feces/urine/menstrual fluids. Therefore the adhesive materials used to attach the device to the human body are particularly effective in providing a strong and stable skin adhesion.

A problem associated with the strong adhesion to the skin of the body adhesive is in positioning and removal. In fact, when positioning the device in correspondence of an excretory orifice, the body adhesive can stick to the hands of the person handling it thus making it difficult to position the device correctly. The adhesive could also stick to the wearer's skin in a wrong position trough accidental contact during the application step. This problem is enhanced when the person positioning the device is not the wearer, but e.g. a nurse wearing latex gloves. Also in the removal phase excessive adhesion to skin might cause pain when removing the article, especially when the skin has hairs on it.

A solution to the above problem can be provided by the articles described in P&G application WO98/28014 and WO98/27914 where disposable absorbent articles having a collection bag and an adhesive flange are described, having adhesive of selected rheology in order to be skin friendly.

Although the articles described in the above patent application are effective in providing a reliable and skin friendly attachment of the flange to the body, there is still room for further improvements in more effectively tailoring the adhesion characteristics of the adhesive flange, and in making the handling of the article easier. Therefore an objective of the present invention is to provide an excreta management device for adhesive attachment to the user's skin comprising a simpler body adhesive structure which can be handled easily in wearing and removing the article and which, while being perfectly adherent to the skin when in use, is easily removed with low or no pain.

Another problem associated with such articles is that suitable body adhesives usually have a gelly appearance and a consistency that when applied in direct contact with the skin causes the users a cold feeling they usually dislike.

Therefore an objective of the present invention is to provide an excreta management device comprising a body adhesive structure where the feeling on skin of the body adhesive surface is more pleasant for the user.

Therefore another objective of the present invention is to provide an excreta management device comprising a body adhesive structure which, when removed after use, leaves no or less residues on skin.

Hence there is a need to provide devices which can be effectively utilized to collect and/or absorb feces, urine, menstrual fluids and the like, which are releasably attached to the human body and thereby prevent leakage and soiling, whilst being easy to handle, not painful to remove, of pleasant appearance and feel and which leave no residues when removed.

### Summary of the Invention

The present invention relates to a disposable excreta management device comprising a means for excreta collection and storage, and a composite body adhesive structure for releasable attachment of the device to the body of a wearer. The composite body adhesive structure comprises:
- a substrate
- a layer of an adhesive material coated onto the substrate
The layer of adhesive material further comprises a pattern of less adhesive areas where the adhesive is inactivated or covered by a second material having a lower adhesion on skin compared to the adhesive material.

### Brief Description of the Drawings

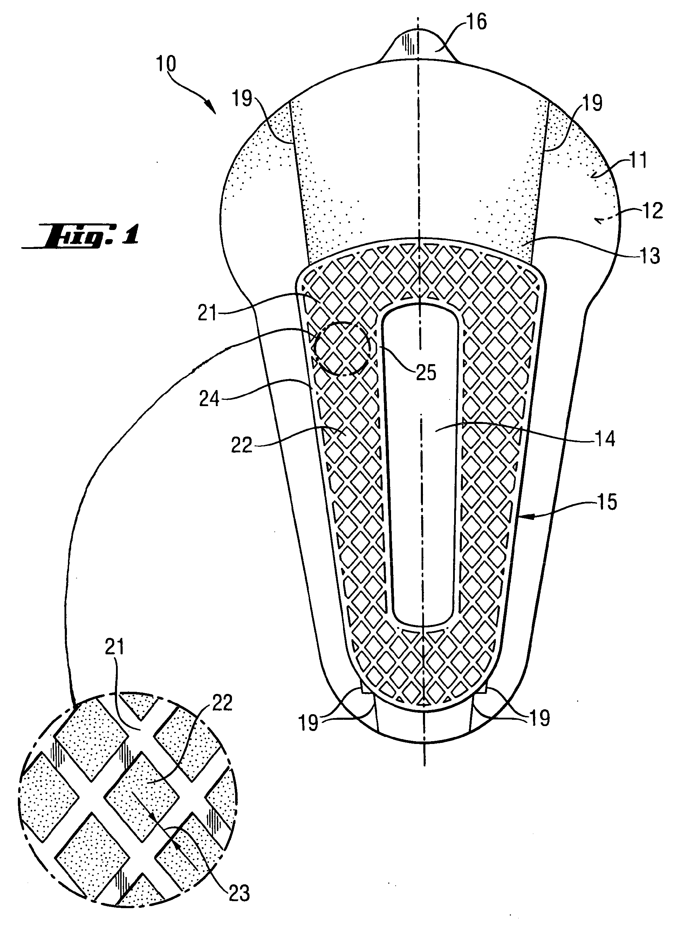

Figure 1 is a plan view of a preferred embodiment of the device according to the present invention with an enlargement of a portion of the composite body adhesive structure having a net-like pattern of less adhesive areas. For easier representation the drawing shows only a portion of the pattern which however interest the whole surface of the composite body adhesive structure.

### Detailed Description of the Invention

All cited references are incorporated herein by reference in their entireties. Citation of any reference is not an admission regarding any determination as to its availability as prior art to the claimed invention.

The definitions of several terms are first provided to assist the reader in understanding the present invention.

The term "comprising" means that other steps and other ingredients which do not affect the end result can be added. This term encompasses the term "consisting of" and "consisting essentially of".

The term "disposable" as used herein, describes devices which generally are not intended to be laundered or otherwise restored or reused (i.e., they are intended to be discarded after a single use and, preferably, to be recycled, composted or otherwise disposed of in an environmentally compatible manner.)

The term "excreta" or "bodily discharges", as used herein, are interchangeable, and includes all discharges released from an excretory orifice of a human body, including fecal materials, urine, menses, and the like, and also e.g. milk or sweat. The term "excretory orifice", as used herein, refers to an orifice which excreta pass through to discharge the excreta from the human body when excretion occurs. Such an excretory orifice includes urethra, vaginal orifice, anus, and the like.

The disposable excreta management devices of the present invention (except for what concerns the inventive pattern of less adhesive areas), are those described in the prior art, for example in US appl. 60/356901, WO00/00132, WO98/28014, WO98/27914, EP 1,104,666 and EP 966,936.

The disposable excreta management devices of the present invention differentiates from those described in the mentioned prior art in that they comprise means for collecting and storing excreta and a composite body adhesive structure for releasable attachment to the skin, which comprises a substrate and a layer of an adhesive material coated onto said substrate. Said layer of adhesive material comprises a pattern of less adhesive areas where the adhesive is inactivated or covered by a second material having a lower adhesion on skin compared to said adhesive material. The adhesion on skin of the second material, in comparison to the adhesion of the adhesive material, can be evaluated with known means, as can be readily determined by the man skilled in the art, such as for example by measuring at standard conditions the peel adhesion on skin of the adhesive material, and of the second material, with a suitable peel adhesion test as it is known in the art, for example according to the test method referred to below. In said test the adhesive material is usually coated onto a substrate, while the second material can be tested as such, if it is already in form of a layer, or when suitably applied onto a substrate. Typically, and preferably, said second material comprises a material which is not adhesive on the skin, as can be readily ascertainable by the man skilled in the art.

Figure 1 shows one preferred embodiment of a disposable excreta management device of the present invention which can be attached to the skin around the excretory orifice, e.g. in the uro-genital area, of a wearer.

The excreta management device 10 shown in Figure 1 has two surfaces; one is a wearer-facing surface 11 and the other is an opposing surface 12. The wearer-facing surface 11 is the surface of the device 10 which is generally oriented toward the wearer when the device 10 is worn. The wearer-facing surface 11 typically at least partially comes in contact with the wearer's skin during use of the device 10. The opposing surface 12 is the surface of the device 10 which is generally oriented away from the wearer when the device 10 is worn, and at least partially toward a garment if a garment is worn. The excreta management device 10 of this preferred embodiment of the present invention comprises means for excreta collection and storage constituted by a flexible bag 13 having an opening 14 whereby excreta are received from the body prior to collection and storage in the bag cavity. The opening 14 is surrounded by an adhesive flange 15 constituting the composite body adhesive structure for releasable attachment of the device to the wearer's body. The adhesive flange 15 comprises a substrate coated with a layer of an adhesive material.

According to this preferred embodiment of the present invention, the pattern of less adhesive areas on the layer of adhesive material is provided with a second material constituted by a non-adhesive nonwoven sheet 21, e.g. a nonwoven or plastic sheet, or laminate threof, suitably perforated in order to define a net-like pattern, which covers the layer of adhesive material on the flange 15, thus leaving the adhesive exposed only in correspondence of the lozenge-shaped holes 22.

The presence of a pattern of less adhesive areas on the adhesive layer of an article according to the present invention allows to tailor the adhesive characteristics of the adhesive material which constitutes the layer without changing the adhesive. In particular, the introduction of a pattern of less adhesive areas, by having a higher or lower percentage of less adhesive areas, allows to tailor exactly the adhesiveness of the adhesive layer in all the parts of the adhesive structure, in order to ensure perfect sealing and comfortable removal, in addition it also allows to have lower or no adhesiveness in selected zones of the adhesive layer like the borders, which help the handling of the device, avoiding it to stick to the hands of the user. The presence of a pattern of less adhesive areas is also beneficial in that it reduces the cold sensation that can be caused by direct contact with the skin of those preferred adhesives having a jelly consistency, namely oilgels and hydrogels. The pattern of less adhesive areas is also effective in reducing the possibility that residues of adhesive are left on the skin upon removal of the device, since it increases the cohesiveness of the adhesive material on its surface actually contacting the skin.

Preferably said pattern extends for the entire surface of the composite body adhesive structure covered by the layer of adhesive material, but in some cases, depending on the application and on the targeted user groups it might be useful to have said pattern of less adhesive areas distributed only on a portion of the layer of adhesive material. This can be useful when a part of said composite body adhesive structure is subjected during use to higher stress. In this case a part of said composite body adhesive structure with no pattern of less adhesive areas can provide a stronger adhesion just where it is needed while maintaining the advantages of the present invention on the rest of the composite body adhesive structure.

In general, when considering only the portion of the layer of adhesive material which, according to the present invention, comprises the pattern of less adhesive areas, said pattern of less adhesive areas has a total surface area which usually represents from 10 to 80%, preferably from 20 to 70%, more preferably from 30 to 60% of the total surface area of said layer of adhesive material interested by said pattern of less adhesive areas.

Said pattern of less adhesive areas can be regular or irregular. Regular patterns are in general easier to make in a controlled way in order ot provide the desired result on the composite body adhesive structure, and are also preferred for aesthetic reasons. For the sake of clarity of the description, suitable patterns of less adhesive areas for these applications will be classified in two classes: "continuous patterns" and "patterns with a plurality of spaced apart discrete less adhesive areas". Nevertheless it should be understood that a composite body adhesive structure according to the present invention may comprise both types of patterns of less adhesive areas on different portions of its layer of adhesive material.

"Continuous patterns" in this case are those which are formed by a number of interconnected less adhesive areas and which define a plurality of spaced apart adhesive areas, in which the adhesive is not covered neither inactivated. Said spaced apart adhesive areas can be in the form of rectilinear or curved stripes, dots, circles, squares, rectangles, triangles, lozenges, regular or irregular polygons in general, spirals or their combination. Preferably said spaced apart adhesive areas have an average surface area of from 0,5 to 400 mm², more preferably from 1 to 100 mm², even more preferably from 5 to 50 mm².

In a preferred embodiment of the present invention, immediately adjacent spaced apart adhesive areas are spaced apart from each other by a distance of not less than 0.5 mm, preferably from 0.5 to 5 mm, more preferably from 1 to 3 mm, which distance hence represents the thickness or width of the interconnected less adhesive areas forming the continuous pattern measured between two immediately adjacent spaced apart adhesive areas.

A preferred case of a "continuous pattern" is that of a regular continuous pattern for example with a net-like structure. In this preferred case the net can have square, rhombic, polygonal or circular holes and a net thread having a thickness of from 0.5 to 5 mm, preferably from 1 to 3 mm. In the preferred embodiment of the present invention represented in Fig. 1, a net like pattern 21 of less adhesive areas is used which defines a plurality of spaced apart adhesive areas of lozenge shape 22, the net having a thread width 23 of about 1.5 mm.

"Patterns with a plurality of spaced apart discrete less adhesive areas" are in this case like the "negative image" of the "continuous patterns". In this case spaced apart discrete less adhesive areas are distributed in a pattern on the surface on the adhesive layer. Said spaced apart discrete less adhesive areas can be in the form of rectilinear or curved stripes, dots, circles, squares, rectangles, triangles, lozenges, regular or irregular polygons in general, spirals or their combination. Preferably said spaced apart discrete less adhesive areas have an average surface area of from 0,5 to 500 mm², preferably from 10 to 400 mm², more preferably from 30 to 300 mm².

In an embodiment of the present invention which is preferred when using "patterns with a plurality of spaced apart discrete less adhesive areas", immediately adjacent spaced apart discrete less adhesive areas are spaced apart from each other by a distance of not less than 0.5 mm, preferably from 0.5 to 5 mm, more preferably from 1 to 3 mm.

Preferred patterns of less adhesive areas according to the present invention are "continuous patterns", particularly regular net-like patterns.

According to the present invention, said pattern of less adhesive areas can be generally obtained by inactivating the adhesive surface in any way known in the art according to said pattern, using e.g. chemical or thermal treatments. Alternatively and preferably the pattern of less adhesive areas is obtained by covering portions of the adhesive layer with a second material which is less adhesive on skin than the adhesive itself.

In the preferred case when the adhesive in the less adhesive areas is inactivated by covering it with a second material, said second material which covers the adhesive can be a plastic or fibrous of powder material. In the case of plastic it is preferably a plastic layer or film, in the case of fibrous materials said second material can be a knitted net, a woven or, preferably, a non woven layer. In some cases also powder materials can be used, which dispersed on selected zones of the adhesive layer according to a desired pattern can make it less adhesive or not adhesive at all. Suitable powder materials for this purpose are all inert powders, talc and zeolites are particularly preferred.

Preferred second materials are plastic films and non woven layers or laminated structures thereof. In the preferred case of "continuous patterns" said second material is preferably a plastic film or a nonwoven layer or a laminate thereof, suitably perforated in order to provide a desired net-like pattern. Alternatively it can be a stretchable net i.e. for example a plastic film or a nonwoven layer which is cut with a multitude of equally spaced, parallel cuts, in a way well known in the art, so that, when stretched, it generates a net and can then be applied onto the layer of adhesive material. Such preferred materials can also provide the plurality of spaced apart discrete less adhesive areas according to the alternative embodiment of the present invention by being suitably shaped and applied with known means onto the layer of adhesive material in order to provide the desired pattern.

Although said second material can have some adhesiveness on skin, it is preferred that said second material is substantially non adhesive on skin.

Any known adhesive structure for releasable attachment to the skin comprising a substrate and a layer of adhesive material coated onto said substrate can be used in the present invention.

Said layer of adhesive material is generally a continuous layer. However in some particular cases it might be preferred that such layer presents some discontinuity, for example it might be applied in stripes, dots or squares. One case in which a discontinuous layer can preferred is when the adhesive is hydrophobic (such as an oilgel) and does not allow the passage of water vapor from the skin. Discontinuities in this case allow the passage of water vapor from the skin to the environment thus making the excreta management device of the present invention more comfortable to wear. In this case, for the purpose of calculating the percentage of the total surface area of the layer of adhesive material occupied by said pattern of less adhesive areas, every single zone where the adhesive layer is continuous and interested by the pattern of less adhesive areas will be considered individually, and the sum of the respective areas made. Known techniques of image analysis could e.g. be used for this scope, and in general for the evaluation of said percentage surface area of the pattern of less adhesive areas.

According to the present invention any medically approved water resistant pressure sensitive adhesive may be used to attach the device to the skin of the wearer. Suitable adhesives for the present invention are described for example EP 1,104,666. Particularly preferred for the present application are hydrogels and oilgels.

According to a preferred embodiment of the present invention said second material has preferably opposite hydrophilicity with respect to the adhesive material. For example when using hydrogels (hydrophilic adhesives) as adhesive material, hydrophobic second materials are preferred (for example a polyethylene material), while when using oilgels (hydrophobic adhesives) as adhesive material, hydrophilic second materials are preferred (for example a cellulosic non woven material). This can help in the management of moisture, e.g. coming from the skin during wear, thus improving the stability of the adhesive in moist or wet conditions.

The adhesion to skin of the layer of adhesive material as such, i.e. not comprising the pattern of less adhesive areas, is preferably between 0,2 and 5 N/cm. The average adhesion of the composite body adhesive structure is preferably from 0.1 to 2 N/cm and in all cases is at least 10%, preferably at least 30%, less than the adhesion of the same adhesive structure without the pattern of less adhesive areas on it.

The adhesion is measured as peel adhesion on skin according to the test described in P&G patent application EP 1,214,896 where it is described a quantitative method to measure peel adhesion on skin of a sample of an adhesive structure. Said method can be applied to a sample of a composite body adhesive structure according to the present invention, and to a respective comparative sample not comprising the pattern of less adhesive areas. The test has to be performed in dry skin conditions and in conditioned room as described in the test methodology.

The average basis weight of the layer of adhesive material can be varied depending on the adhesive type and on the desired end use of the product, as it is known in the art. For the oilgel or hydrogel adhesives, preferred in the present invention, the basis weight is preferably < 2000 g/m² more preferably <1500 g/m² even more preferably 100-1500 g/m².

Suitable substrates for the adhesive structure of the present invention are described in EP 1,104,666. Preferred substrates for the present invention are open cell foam layers having a thickness from 0.5-10 mm.

In the preferred embodiment of the present invention illustrated in Fig 1, where the disposable excreta management devices comprises as means for excreta collection and storage, a bag having an aperture, said aperture being surrounded by said composite adhesive structure for releasable attachment to the skin, the bag can be constituted by the backsheet and the topsheet of the article, its aperture being in the topsheet, and in this case the topsheet is preferably water impervious. Alternatively said bag can be constituted by a single layer of material. Typically the bag is made of liquid impervious material, which optionally can be also moisture vapour or gas permeable.

Alternatively, disposable excreta management devices according to the present invention can comprise disposable absorbent articles for direct attachment to the user's body as they are known in the art, i.e. comprising a liquid permeable topsheet, a liquid impermeable backsheet, and an absorbent core therebetween, of course with the composite body adhesive structure of the present invention. Such alternative devices can find use as sanitary napkins, adult incontinence devices, pantyliners, baby diapers, as well as breast or sweat pads.

The composite body adhesive structure comprising the substrate and the layer of adhesive material having the pattern of less adhesive areas can be totally or partially attached to the topsheet of the disposable excreta management device, wherein "topsheet" also indicates the body facing surface of a bag, as explained above. When the composite body adhesive structure is substantially completely attached to the topsheet it may be preferred for some applications that the substrate of said composite body adhesive surface is the topsheet of the article or the body facing portion of the bag itself.

In an alternative embodiment said composite body adhesive structure is not completely attached to the topsheet, typically the body facing surface of the bag, such as for example when said composite body adhesive structure is in the form of a circular or oblong flange which is attached to the topsheet along a line close to the border of its aperture. This arrangement is particularly useful when a certain degree of freedom of the device, namely of the bag, with respect to the body of the user is required, and this is better provided by a flange which is not completely attached to the bag.

In the preferred case where the disposable excreta management article of the present invention comprises a bag, conventionally the composite body adhesive structure, with its adhesive material, will completely surround the aperture 14. In principle it is, however, also possible and maybe desirable depending on the targeted user group, that the adhesive structure, or at least the adhesive itself, is not completely surrounding the aperture. For example in the use of a fecal, urine, or menstrual management device, a "U" shape of the composite body adhesive structure and/or of the adhesive may be selected where the open part of the composite body adhesive structure and/or of the adhesive is directed towards the front of the wearer during use so as not to create difficulties in the context of the pubic hair primarily growing in the front of the genital area of the targeted user group.

According to a particularly preferred embodiment of the present invention, the layer of adhesive material coated onto the substrate in the composite body adhesive structure has a pattern of less adhesive areas which further comprises an area along the external perimeter of the composite body adhesive structure, identified in Figure 1 with the reference number 24. When the composite body adhesive structure is comprised in a preferred device having a bag with an aperture, wherein the composite body adhesive structure substantially surrounds said aperture, the pattern of less adhesive areas also preferably comprises an area along the internal perimeter of the composite body adhesive structure, identified in Figure 1 with the reference number 25, wherein said internal perimeter corresponds to the edge of the composite body adhesive structure which runs along the border of the aperture.

According to a further alternative embodiment of the present invention, the layer of adhesive material coated onto the substrate has a lower thickness along the external perimeter of the composite body adhesive structure, and preferably also along the internal perimeter when the composite body adhesive structure is incorporated in a device having a bag with an aperture, compared with the thickness of the layer of adhesive material in the remaining portions of the composite body adhesive structure. Most preferably, the lower thickness adhesive layer along the external and/or internal perimeter of the composite body adhesive structure is combined with the pattern of less adhesive areas also comprising the area along said external and/or internal perimeter.

The above features generally allow an easier handling of the device avoiding it to stick to hands or latex gloves when it is being applied. They also help reducing possible residues of body adhesive left on skin upon removal.

The composite body adhesive structure of the present invention can be further modified in any way known in the art to make it more suitable for its intended use, for example said composite body adhesive structure may also have non adhesive lobes for grasping and holding the structure, e.g. when applying or removing the device.

Alternatively, the external and/or the internal perimeter of said composite body adhesive structure can be not linear, but provided as a sinusoidal wave edge which has been found to provide additional comfort, especially upon removal of the article.

The flexible bag 13, as used in the preferred embodiment of the present invention, is a flexible receptacle for the containment of discharged excreta, such as fecal materials, urine, menses or the like. The bag 13 can be provided in any shape or size depending on the intended use thereof, i.e., whether the device is intended for bedridden patients or active patients suffering from incontinence or for menstrual usage. For example, elongated bags which are principally tubular or rectangular are typically utilized by bedridden patients and elderly incontinence sufferers. For more active wearers such as infants or adults, the excreta management device 10 should preferably be anatomically shaped such that the device 10 follows the contours of the body and can be worn inconspicuously by the wearer under normal garments. Particularly, preferred shapes are three-dimensional shaped bags such as cubic shaped bags, spherical shaped bags, conical (or truncated conical) shaped bags, pyramidal (or truncated pyramidal) shaped bags, tetrahedral (or truncated tetrahedral) shaped bags, cylindrical shaped bags or the like. Further, when the bag is not expanded, the bag may have a substantial circular, oval, square, rectangular, polygonal shape.

The bag 13 is preferably designed to provide sufficient volume for excreta under a variety of wearing conditions, e.g., when the device 10 is worn by active wearers (i.e., not bedridden wearers). The bag can be also optionally provided with folds such as for example Z-folds 19 as indicated in Fig. 1, which upon unfolding in use allow further internal space. The bag 13 is designed to safely contain any entrapped material, typically it will be liquid impermeable, yet it may be breathable. The bag 13 is designed to having sufficient strength in order to resist rupturing in use, e.g., when pressure on the bag 13 is exerted in typical wearing condition such as sitting.

The bag 13 may be made from a unitary piece of material or fro m a number of separate pieces of material, which may be identical or different and which are sealed at their respective peripheries, depending on the shape of the bag 13 required. In a preferred embodiment the bag 13 constituting the excreta management device 10 is made from a single web sheet.

The bag 13 can comprise one or multiple layers, preferably two or three layers. The layer on the inside of the bag, which will typically at least partially come in contact with excreta, is called the inner layer. The outermost layer of the bag 13, which will typically at least partially come in contact with the skin of a wearer and the garments of the wearer, is called the outer layer. The layer of the bag may be provided from any material such that the bag is liquid impervious. The layer may in particular comprise any material such as a nonwoven or a polymeric film. In a preferred embodiment, the layer may be formed from a laminate comprising a nonwoven layer and a polymeric film. The outer layer of the bag 13 is preferably provided with a nonwoven layer. The nonwoven outer layer presents a compliant surface to the skin of a wearer and thus greatly improves skin healthiness. In one preferred embodiment, the bag 13 comprises two layers, which comprises a nonwoven layer as the outer layer and a film as the inner layer. Alternatively, the bag 13 may comprise three layers; one film layer and two nonwoven layers. Preferably, the film may be interposed between the two nonwoven layers. This sequence of layers results in a closed fibrous structure, which has a particularly pleasing sensation on contact with the skin of a wearer.

Suitable nonwoven layers for the bag 13 may comprise felt fabrics, spunlaced fabrics, fluid jet entangled fabrics, air-laid fabrics, wet-laid fabrics, dry-laid fabrics, melt-blown fabrics, staple fiber carding fabrics, spunbonded fabrics, stitch-bonded fabrics, apertured fabrics, combinations of the above or the like. The nonwoven layer or the nonwoven layers constituting the bag 13 may be hydrophobic or hydrophilic. For example, if the bag 13 comprises a film layer, the nonwoven layers may be hydrophilic or hydrophobic. If the bag 13 does not comprise a film layer, preferably at least one nonwoven layer is hydrophobic. It may still be desirable to make both nonwoven layers hydrophobic to ensure that the bag is liquid impervious. Typically, the nonwoven layer is treated with a surface active material, such as a fluorchemical or other hydrophobic finishings, to provide the requisite hydrophobicity. The nonwoven layer, however, may equally be treated with coatings of liquid impervious materials such as hot-melt adhesives or coatings of silicone or other hydrophobic compounds such as rubbers and vegetable and mineral waxes or it may be physically treated using nano-particulates or plasma coating techniques, for example. The nonwoven layer can also be treated with agents to improve the tactile perceivable softness. The agents include but are not limited to vegetable, animal or synthetic oils, silicone oils and the like. The presence of these agents is known to impart a silky or flannel-like feel to the nonwoven layer without rendering it greasy or oily to the tactile sense of the wearer. Additionally, surfactant material, including anionic, non-anionic, cationic and non-cationic surfactants, may be added to further enhance softness and surface smoothness. Furthermore, the nonwoven layer may be impregnated with a lotion to provide desirable therapeutic or protective coating lotion benefits. The lotion coating is transferable to the skin of a wearer by normal contact and wearer motion and/or body heat. Generally, mineral oil in the form of a lotion is recognized as being effective in imparting a soothing, protective coating to the skin of a wearer. It is also possible to impregnate the nonwoven layer with a solid oil phase of cream formulation or to incorporate into the nonwoven layer an array of pressure- or thermal- or hydrorupturable capsules containing for example, baby oil.

Suitable film materials for the bag 13 may comprise a thermoplastic material. The thermoplastic material can be selected from among all types of polyolefins especially polyethylene, polypropylene, amorphous polyolefins, and the like; material containing meltable components comprising fibers or polymeric binders including natural fibers such as cellulose - wood pulp, cotton, jute, hemp; synthetic fibers such as fiberglass, rayon, polyester, polyolefin, acrylic, polyamid, aramid, polytetrafluroethylene metal, polyimide; binders such as bicomponent high melt/low melt polymer, copolymer polyester, polyvinyl chloride, polyvinyl acetate/chloride copolymer, copolymer polyamide, materials comprising blends wherein some of the constituent materials are not meltable; air and vapour permeable materials including microporous films such as those supplied by EXXON Chemical Co., III, US under the designation EXXAIRE or those supplied by Mitsui Chemical Co., Japan under the designation ESPOIR NO; and monolithic breathable materials such as Hytrel™ available from DuPont and Pebax™ available from ELF Atochem, France. In a preferred embodiment, a film which is comprised in any layer is preferably permeable to gases such as air and to vapour such as water vapour in order to avoid the problem of entrapment and condensation of moisture vapour given off by the body of the wearer and thus, the hot, clammy and uncomfortable conditions after a short period of use.

The bag 13 may further contain an absorbent material therein. The absorbent material may be positioned inside the bag 13 in any suitable manner. For example, the absorbent material may be loosely arranged within the bag 13 or may be secured to the inner side of the bag 13. Any known techniques for securing absorbent material to nonwoven and film substrates may be used to secure the absorbent material to the inner side of the bag 13. The absorbent material may also be arranged to have any desired shape or configuration (e.g., rectangular, oval, circular, etc.). The absorbent material may comprise any material which is capable of absorbing and retaining discharged body fluids. The absorbent material may comprise a wide variety of liquid-absorbent materials commonly used in disposable diapers and other absorbent articles such as comminuted wood pulp, which is generally referred to as airfelt. Examples of other suitable absorbent materials include creped cellulose wadding; meltblown polymers, including coform; chemically stiffened, modified or cross-linked cellulosic fibers, synthetic fibers such as crimped polyester fibers; peat moss; tissue, including tissue wraps and tissue laminates; absorbent foams; absorbent sponges; superabsorbent polymers; superabsorbent hydrogel-forming polymeric material; absorbent gelling materials; or any other known absorbent material or combinations of materials or mixtures of these. The configuration and construction of the absorbent component may also be varied (e.g., the absorbent component may have varying caliper zones (e.g., profiled so as to be thicker in the center), hydrophilic gradients, superabsorbent gradients, or may comprise one or more layers or structures.

An assistant tab can be disposed anywhere on the article and may assist the user in expand the bag, in positioning or in removing the article as described in US appl. 60/356901. For example as illustrated in Fig. 1, an assistant Tab 16 disposed at an edge of the device 10, The assistant tab 16 is particularly useful for expanding the bag, typically after application to the body, and preferably in combination with the folds 19.

While particular embodiments of the present invention have been illustrated and described, it would be obvious to those skilled in the art that various other changes and modifications can be made without departing from the invention. It is therefore intended to cover in the appended claims all such changes and modifications that are within the scope of this invention.

Articles according to the present invention can be advantageously used in all excreta management devices which are applied directly to the body of the user. For example adult urinary or fecal incontinence devices for males or females, baby diapers, sanitary napkins, catamenial, panty liners, breast or sweat pads.

### Example.

A disposable excreta management device according to the present invention can be made as drawn in Figure 1, by covering an adhesive hydrogel flange of a bag-flange urinary incontinence device with a laminated structure comprising a polyethylene film and a nonwoven layer, wherein the nonwoven layer is oriented towards the user's body, said laminated structure being perforated in order to provide a net pattern with regular lozenge shaped holes having an area of around 25 mm² each. The net threads are 1.5 mm wide. The net pattern also comprises a continuous thread along the external and internal perimeter of the flange. The flange constitutes, in this preferred example, the composite body adhesive structure of the present invention.

## Claims

1. A disposable excreta management device comprising means for excreta collection and storage, and a composite body adhesive structure for releasable attachment of said device to the body of a wearer, said structure comprising:
- a substrate
- a layer of an adhesive material coated onto said substrate
said device being **characterized in that** said layer of adhesive material comprises a pattern of less adhesive areas where the adhesive is inactivated or covered by a second material having a lower adhesion on skin compared to said adhesive material.

2. A disposable excreta management device according to claim 1 wherein said pattern of less adhesive areas has a total surface area which represents from 10 to 80%, preferably from 20 to 70%, more preferably from 30 to 60% of the total surface area of said layer of adhesive material interested by said pattern of less adhesive areas.

3. A disposable excreta management device according to any preceding claim wherein said second material is a plastic or fibrous or powder material.

4. A disposable excreta management device according to any preceding claim wherein said pattern is a continuous pattern of interconnected less adhesive areas.

5. A disposable excreta management device according to claim 4 wherein said pattern is a regular pattern, preferably in the form of a net-like pattern.

6. A disposable excreta management device according to claim 4 or 5 wherein said pattern defines a plurality of spaced apart adhesive areas, said spaced apart adhesive areas being **characterized in that** their average surface area is from 0,5 to 400 mm², preferably from 1 to 100 mm², more preferably from 5 to 50 mm².

7. A disposable excreta management device according to claim 6, wherein immediately adjacent spaced apart adhesive areas are spaced apart from each other by a distance of not less than 0.5 mm, preferably from 0.5 to 5 mm, more preferably from 1 to 3 mm.

8. A disposable excreta management device according to claims 1-3 wherein said pattern comprises a plurality of spaced apart discrete less adhesive areas, preferably in the shape of dots or of rectilinear or curved stripes.

9. A disposable excreta management device according to claim 8 wherein said spaced apart less adhesive areas are **characterized in that** their average surface area is from 0,5 to 500 mm², preferably from 10 to 400 mm², more preferably from 30 to 300 mm².

10. A disposable excreta management device according to claim 3 wherein said plastic or fibrous material is a perforated plastic or nonwoven layer or a stretchable net.

11. A disposable excreta management device according to any preceding claim wherein said second material is substantially non adhesive on skin.

12. A disposable excreta management device according to any preceding claim wherein said means for excreta collection and storage comprises a bag, said bag having an aperture, said aperture being surrounded by said composite adhesive structure for releasable attachment of said device to the uro-genital area of a wearer.

13. A disposable excreta management device according to claim 12 wherein said composite adhesive structure has an external perimeter and an internal perimeter, said internal perimeter substantially corresponding to said aperture in said bag and wherein said pattern of less adhesive areas comprises an area along said external perimeter and, preferably, an area along said internal perimeter.

14. A disposable excreta management device according to claim 13, wherein said layer of adhesive material is thinner along said external perimeter, and preferably along said internal perimeter, of said composite adhesive structure than in the remaining portions of said layer of adhesive material.
